# FASCICULE DE BREVET EUROPEEN

(11) **EP 1 541 118 B1**
(45) Date de publication et mention de la délivrance du brevet: **24.01.2007**
(21) Numéro de dépôt: 04292890.3
(22) Date de dépôt: 06.12.2004
(51) Int. Cl.: A61K 8/84, A61K 8/90, A61Q 5/12

(54) **Composition cosmétique capillaire contenant un nano-objet de forme allongée en polymère synthétique réticulé, procédé mettant en oeuvre cette composition et utilisations**
Haarkosmetische Zusammensetzung enthaltend ein langgestrecktes NANOOBJEKT aus einem vernetzen, synthetischen Polymer, Verfahren zur Anwendung derselben und Verwendungen
Hair cosmetic composition containing an elongated NANO-OBJECT from a cross-linked, synthetic polymer, process for applying this composition and uses

(30) Priorité: 11.12.2003 FR 0314541
(43) Date de publication de la demande: 15.06.2005
(73) Titulaire: L'ORÉAL, 75008 Paris (FR)
(72) Inventeur: Mathonneau, Estelle, 75010 Paris (FR); Rollat, Isabelle, 75017 Paris (FR); Mougin, Nathalie, 75011 Paris (FR)
(74) Mandataire: Dossmann, Gérard

(56) Documents cités:
- EP-A- 1 371 354
- S. STEWART, G. LIU: "Block Copolymer Nanotubes" ANGEW. CHEM. INT. ED., vol. 39, no. 2, 2000, pages 340-344, XP002284711
- JOSE RAEZ, RALUCA BARJOVANU, JASON A. MASSEY, MITCHELL A. WINNIK, IAN MANNERS: "Self-Assembled Organometallic Block Copolymer Nanotubes" ANGEW. CHEM. INT. ED., vol. 39, no. 21, 2000, pages 3862-3865, XP002284712

## Description

La présente invention a pour objet une composition cosmétique pour le traitement cosmétique des fibres kératiniques, en particulier des fibres kératiniques humaines telles que les cheveux, comprenant dans un milieu cosmétiquement acceptable, au moins un nano-objet de forme allongée en polymère synthétique réticulé, elle a aussi pour objet les procédés de traitement cosmétique des fibres kératiniques qui mettent en oeuvre cette composition pour conférer du volume à la coiffure, et les utilisations de cette composition cosmétique capillaire pour conférer du volume à la coiffure.

Les produits de coiffage destinés à améliorer le volume de la coiffure les plus répandus sur le marché sont à base de polymères filmogènes. A l'application, ces produits permettent le plus souvent d'obtenir l'effet recherché, cependant l'effet obtenu s'estompe rapidement et disparaît totalement au premier shampooing. Ces produits présentent aussi l'inconvénient d'altérer les cheveux et de leur conférer un toucher chargé.

Une autre technique pour améliorer le volume de la coiffure consiste à effectuer un traitement de mise en forme permanente des fibres kératiniques, ces permanentes consistent à appliquer sur les fibres kératiniques un agent réducteur, à les soumettre à une mise sous tension mécanique généralement au moyen de rouleaux puis à leur appliquer un agent oxydant. Cette technique permet d'améliorer durablement le volume de la coiffure mais elle présente les inconvénients de modifier la forme de la coiffure et le niveau de la frisure, et aussi de dégrader la fibre kératinique.

De manière surprenante et avantageuse, la Demanderesse a découvert que l'utilisation de nano-objets de forme allongée en polymère synthétique réticulé dans une composition cosmétique permet d'augmenter le volume de la coiffure de façon durable.

Ainsi la présente invention concerne une composition cosmétique pour le traitement cosmétique des fibres kératiniques, en particulier des fibres kératiniques humaines telles que les cheveux, comprenant dans un milieu cosmétiquement acceptable, au moins un nano-objet de forme allongée en polymère synthétique réticulé et un additif cosmétique selon la revendication 1.

Des nanotubes en polymère synthétique sont déjà connus de l'art antérieur, en particulier les articles S. Stewart *et al.* « Block Copolymer Nanotubes » Angew. Chem. Int. Ed, vol. 39, n° 2, 2000, pages 340-344 et Jose Raze *et al.* « Self-Assembled Organometallic Block Copolymer Nanotubes », Angew. Chem. Int. Ed, vol 39, n° 21, 2000, pages 3862-3865 concernent la synthèse de nanotubes en polymère synthétique dans des solvants classiques ; ces documents ne mentionnent cependant pas une utilisation de ces nanotubes avec des additifs cosmétiques dans le domaine cosmétique capillaire.

En outre la demande de brevet EP 1 371 354 divulgue un autre procédé destiné à conférer du volume aux cheveux, ce procédé met en oeuvre des compositions cosmétiques comprenant des nanotubes constitués d'au moins un élément appartenant aux groupes II_{A}, III_{A}, IV_{A}, V_{A}, VIII, I_{B}, II_{B}, III_{B}, VI_{B}, VII_{B}.

La composition selon l'invention présente une bonne affinité pour les fibres kératiniques. Cette composition permet une amélioration sensible du volume de la coiffure qui peut être rémanente à un ou plusieurs shampooings.

En outre, de par leur nature, les compositions selon la présente invention ne contiennent pas d'agents dégradants pour les fibres kératiniques. Les fibres kératiniques ne sont, par conséquent, pas altérées par les applications répétées des compositions selon l'invention, et leur toucher n'est pas modifié.

Ces compositions possèdent également de bonnes propriétés cosmétiques en termes de douceur, légèreté de la coiffure.

D'autres objets, caractéristiques, aspects et avantages de l'invention apparaîtront encore plus clairement à la lecture de la description et des exemples qui suivent.

Par « nano-objet de forme allongée en polymère réticulé » au sens de la présente demande, on entend tout objet tridimensionnel constitué d'un ou plusieurs polymères réticulés dont la dimension selon un des axes (longueur) est strictement supérieure aux dimensions selon les deux autres axes (section).

Ces nano-objets de forme allongée en polymère réticulé sont notamment des nanotubes présentant une section circulaire, elliptique ou hexagonale de diamètres externes ou d'axes de préférence compris entre 0,1 et 200 nm, ou encore des hélices de diamètre externe de préférence compris entre 0,1 et 200 nm.

La longueur de ces nano-objets de forme allongée en polymère réticulé est de préférence comprise entre 10 nm et 10µm.

Le rapport entre la longueur et la section du nano-objet est strictement supérieur à 1, de préférence supérieur à 2 et de manière encore plus préférée supérieur à 3, ce rapport étant de préférence inférieur à 100 000.

Le polymère utilisé pour obtenir le nano-objet est un polymère synthétique.

Par « polymère synthétique » au sens de la présente demande, on entend un polymère obtenu par synthèse chimique ou électrochimique (polymérisation radicalaire, polycondensation, polymérisation par ouverture de cycle, polymérisation par métathèse). La réticulation peut se faire par voie chimique ou sous l'action du rayonnement photochimique comme par exemple sous l'action des UV ou de la température.

Ce polymère peut-être un homopolymère ou un copolymère. Conviennent tout particulièrement à l'invention, les homopolymères ou copolymères dérivant de la polymérisation radicalaire de monomères comprenant des motifs éthyléniques, vinyliques, allyliques, (méth)acrylates et/ou de (méth)acrylamides et dérivés.

Ainsi conviennent les copolymères vinyliques/(méth)acrylates, vinyliques/(méth)acrylamides, vinyliques/(méth)acrylates/ (méth) acrylamides, oléfiniques/vinyliques et les (méth)acrylates/(méth)acrylamides.
■ A titre illustratif de ces polymères, on peut plus particulièrement citer les homopolymères ou copolymères à base d'acétate de vinyle, de styrène, de vinylpyrrolidone, de vinylcaprolactame, de (méth)acrylate de stéaryle, de (méth)acrylate de lauryle, de laurate de vinyle, de (méth)acrylate de butyle, de (méth)acrylate d'éthylhexyle,
■ d'acide crotonique, d'acide(méth)acrylique, de styrène sulfonate de sodium, de diméthyldiallylamine, de vinyipyridine, de (méth)acrylate de diméthylaminoéthyle, de diméthylaminopropyl(méth)acrylamide et leurs sels.

Il peut également s'agir de :
- polycondensats de polyuréthanne et/ou polyurées, de polyesters aliphatiques, polyamides aliphatiques ou de leurs copolymères, comme par exemple polyuréthanne/urée et polyester/amide,
- polymères obtenus par ouvertures de cycles, comme les polyéthers de type polyoxyde d'éthylène, polyoxyde de propylène, les polylactides et leurs copolymères polyoxyde d'éthylène/ polyoxyde de propylène ; les polyesters comme la polycaprolactone ; les polyoxazolines tels que la poly(2-méthyloxazoline) ou la poly(2-éthyloxazoline),
- (co)polymères de siloxane, par exemple polydiméthylsiloxane (PDMS), polyméthylphénylsiloxane,
- polymères obtenus par métathèse comme le poly(norbornène), ou ses copolymères et,
- de polymères obtenus par polymérisation cationique tels que les polyvinylalkyléthers tel que les polyvinylméthyléthers,
- ou de copolymères de ces différents types de polymères comme par exemple les polysiloxane/polyoxyde d'éthylène.

Ces polymères peuvent, le cas échéant, être fonctionnalisés de manière à leur conférer un caractère soluble ou dispersible dans l'eau et/ou l'éthanol, ou dans les huiles carbonées, esters, fluorées, silicones.

Avantageusement, l'homopolymère est choisi dans le groupe formé par le polypyrrole, le polyaniline, le polyéthylènedioxythiophène, le polyméthylméthacrylate, le polytétrafluoroéthylène, le poly-L-lactide/palladiumacétate, le poly(p-xylène), le [2.2]paracyclophane polymérisé, le 1,4-quinodiméthane polymérisé, le polystyrène, le polypropylène, le poly-(p-phénylene benzo-bis-oxazole), le polyaryleneethynylène.

Avantageusement, le copolymère est choisi dans le groupe formé par les polystyrène-*b*-poly(2-cinnamoylethylmethacrylate) (PS-*b*-PCEMA), polyacide-acrylate-*b*-poly(2-cinnamoylethylmethacrylate) (PAA-*b*-PCEMA), polybutylacrylate-*b*-poly(2-cinnamoylethylmethacrylate) (PBtA-*b*-PCEMA), polyimine-*b*-poly(2-cinnamoyl-ethylmethacrylate) (PI-*b*-PCEMA).

La liste qui suit est donnée à titre indicatif, elle présente des exemples de nano-objets en polymère synthétique connus dans l'art antérieur et qui peuvent être utilisés dans les compositions selon la présente invention. Cette liste ne doit en aucun cas être interprétée comme une limitation de l'invention.

Dans la liste qui suit les nano-objets en polymère sont regroupés en fonction du procédé de synthèse utilisé pour les obtenir.

Les cinq premières techniques présentées ci-dessous peuvent être utilisées pour préparer des nano-objets en homopolymère ou en copolymère.

### a) Par polymérisation dans les pores d'une membrane.

Ce mode de synthèse permet d'obtenir des nano-objets creux ou pleins. Selon cette technique la forme est figée par la réticulation.

Cette technique a permis l'obtention de nanotubes de polypyrrole (J.Duchet, R.Legras, S.Demoustier-Champagne, *Synth. Met.,* **98** (1998) 113 ; S.Demoustier-Champagne, J.Duchet, R.Legras, *Synth. Met.,* **101** (1999) 20 ; VP.Menon, J.Lei, CR Martin, *Chem. Matr.,* **8** (1996) 2382), de polyaniline (Z.Cai, J.Lei, W.Liang, V.Menon, C.R.Martin, *Chemical Materials,* **3,** (1991), 960 ; J.Duchet, R.Legras, S.Demoustier-Champagne, *Synthetic Metals,* **98** (1998)113), de polyéthylène dioxythiophène (J.L.Duval, P.Rétho, G.Louarn, C.Godon, C.Marhic, S.Demoustier-Champagne, S.Garreau, *Matériaux*, (2002)).

Les procédés de synthèse de nano-objets sont décrits dans les références bibliographiques citées dans la présente demande.

Ces synthèses se font par une polymérisation par oxydation soit par voie électrochimique, soit en utilisant un agent oxydant (voie chimique) sur différents types de membranes (« Template synthesis on track-etched membranes »). Ce mode de synthèse est décrit dans les documents suivants : S.Demoustier-Champagne, E.Ferain, R.Legners, C.Jérôme, R.Jérôme, *Eur. Polym. J*., **34** (1998) 1767 ; S.Demoustier-Champagne, P-Y Staveux, *Chem. Mater.,* **11** (1999) 829 ; C.R.Martin, Science, **266** (1994) 1961-1966.

Le procédé de synthèse le plus utilisé est le procédé par voie électrochimique dans les pores d'une membrane de polycarbonate recouverte d'or (anode). En contrôlant le temps de polymérisation, on peut obtenir des tubes à paroi fine ou épaisse.

La membrane est plongée dans une solution comprenant le monomère et du LiClO₄, la membrane recouverte d'or et l'électrode de référence sont mises sous tension pour réaliser la polymérisation. Tous les monomères pouvant polymériser par voie oxydante et présentant au moins une fonction solubilisante peuvent être utilisés. Par « fonction solubilisante », on entend que le monomère comprend une fonction qui le rend soluble dans le milieu de polymérisation.

La taille (longueur, diamètre, épaisseur) des nano-objets dépend de la taille des pores de la membrane utilisée, du monomère et du temps de polymérisation. R.V.Parthasarathy, C.R.Martin, *Chem. Mater.,* 6 (1994) 1627 a montré qu'avec le polypyrrole un tube épais est rapidement formé, avec le polyaniline, la polymérisation est plus lente et conduit à la formation de tubes fins.

Selon cette technique, une fois le nano-objet formé, la membrane est éliminée par dissolution dans un solvant tel que le dichlorométhane mélangé à un tensio-actif comme le dodécylsulfate. Le mélange est ensuite soumis à ultrasons pendant une heure pour obtenir une élimination complète du polycarbonate.

Le type de membrane utilisée n'est pas critique, elle doit cependant pouvoir se dissoudre dans un solvant approprié.

Une variante consiste en la technique de « melt weeting of macroporous templates» décrite dans les documents suivants : M.Steinhart, J.Wendorff, de Philips université à Marburg, de R.Wehrspohn, U.M.Gôsele de Max Plank Institute de Wehrspohn et U.Gösele du Max Plank Institute of Microstructure Physics, *Science* (296), 1997 (2002) ; S.Demoustier-Champagne, J.Duchet, R.Legras, *Synthetic Metals,* 101 (1-3), (1999) 20-21.

Cette variante a permis l'obtention de nano-objets en polytétrafluoroéthylène (PTFE), polyméthylméthacrylate(PMMA), poly-L-lactide/palladiumacetate. La membrane utilisée présente une taille de pores très petite, cette membrane est généralement en alumine, silicone oxydée ou en verre. Les pores de cette membrane sont imprégnés par un liquide contenant le polymère, une fois le nano-objet formé, la membrane est dissoute dans une solution de KOH.

### b) Par polymérisation autour d'un support

Cette technique permet d'obtenir des nano-objets généralement creux, selon cette technique la forme est figée par la réticulation.

Selon ce mode de synthèse, le monomère utilisé pour la polymérisation doit avoir au moins une fonction qui permette la polymérisation et de préférence une fonction qui favorise l'interaction dudit monomère avec le support par liaisons de Van der Waals, liaisons hydrogène, liaisons ioniques, liaisons covalentes, liaisons n-π...

La taille (longueur, diamètre, épaisseur) des nano-objets dépend du support (section, longueur), du monomère constituant le nano-obj et, de la quantité de monomère, et du temps de polymérisation.

Les supports sont de forme allongée, ils sont généralement choisis parmi :
- les nanofibrilles naturelles de tout type : cellulose, protéine, soie...
- les nanofibres synthétiques (polyamide par exemple)
- les nanotubes de carbone fonctionnalisé (J.Chen, *Science,* 282 (1998) 95-98) ou non (par des groupements carboxyliques, des fonctions hydrophobes, des amides grasses de formule CONH-4-C₆H₄(CH₂)₁₃CH₃ par exemple)
- les nanotubes de type BₓC_{y}N_{z} (Z.Weng-Sieh, *Physical Review B*, 16 (1995) 11229-32 ; E. Hernandez, C.Goze, P. Bernier, A.Rubio, *Physical Review Letter,* 80(20) (1998) 4502-05)

Les utilisations de fibres comme support de synthèse « fiber template support » ont été présentées dans les documents suivants : H.Hou, Z.Jun, A.Reuning, A.Schaper, J.Wendorff, A.Greiner, *Macromolecule,* **35(7)**, (2002) 2429-31 ; H.Dong, W.E.Jr. Jones, *Polymeric Materials Science and Engineering,* **87** (2002) 273-4.

Des polyamides 4/6 de quelques dizaines de nanomètres de diamètre (obtenus par electrospinning d'une solution de PA/HCOOH à 8% en présence de pyridine), des fibres de poly(L-lactide) avec un diamètre un peu plus important (obtenues par electrospinning d'une solution de PLA(1,5%)/H₂Cl₂ en présence de Pd(OAc)₂ ont servi de support pour l'obtention de nanotubes de polymères tels que le poly(p-xylène), le [2.2]paracyclophane (dimère) et le 1,4-quinodiméthane (monomère). Les supports sont éliminés par extraction au solvant.

Les documents S.Kumar, T.D. Dang, F.E.Arnold, A.R.Bhattacharyya, B.G.Min, X. Zhang, R.A. Vaia, C. Park, W.W.Adams, R.H.Hauge, R.E. Smalley, S.Ramesh, P.A.Willis, *Macromolecules,* **35** (2002) 9039-43 ; R.Andrews, D.Jacques, A.M.Rao, T.Rantell, F.Derbyshire, Y.Chen, J.Chen, R.C. Haddon, *Appl. Phys. Lett.,* **75** (1999) 1329 ; D.Quin, E.C.Dickey, R.Andrews, T.Rantell, *Appl.Phys. Lett.,* **76** (2000) 20 ; R.Haggenmueller, H.H.Gommans, AG. Rinzler, J.E. Fischer, K.I.Winey, *Chem. Phys. Lett.,* **330** (2000) 219 ; S.Kumar, H.Doshi, M.Srinivasrao, J.O.Park, D.Aschiraldi, *Polymer,* **43** (2002) 1701 décrivent des synthèses de nano-objets par polymérisation autour d'un nanotube de carbone.

### c) Par polymérisation autour d'une forme obtenue par un tensio-actif.

Cette technique permet obtenir des tubes généralement creux, selon cette technique la forme est figée par la réticulation.

La taille (longueur, diamètre, épaisseur) des nanotubes dépend du tensio-actif, du monomère constituant le nanotube, de la quantité de monomère, et du temps de polymérisation.

Cette technique a permis l'obtention de nanotubes de polypyrrole. (J.Jang, H.Yoon, *Chem.Comm.,* (2003) 720-21), elle met en oeuvre une émulsion inverse dans un solvant apolaire. Le tensio-actif utilisé pour cette mise en oeuvre est le bis(2-éthylhexyl)sulfosuccinate de sodium dans l'hexane, ce tensio-actif forme une structure micellaire inversée avec des groupes hydrophiles à l'intérieur de la structure micellaire. Le polypyrrole se polymérise sur l'extérieur du tube. Une extraction au solvant du tensio-actif permet obtenir des tubes de polypyrrole.

### d) Par passage d'une solution de polymères dans les pores d'une membrane.

Cette technique permet d'obtenir des tubes généralement pleins, selon cette technique la forme n'est pas figée puisqu'elle peut être perturbée par l'ajout d'un solvant ou d'un additif.

La taille (longueur, diamètre, épaisseur) des nanotubes dépend de la taille des pores de la membrane, et du ou des polymères constituant le nanotube.

Le monomère utilisé pour la polymérisation doit avoir des groupements fonctionnels favorisant l'auto assemblage (par liaisons n-π, liaisons hydrogène, par interaction acide-base, par interaction dipole-dipole...)

Cette technique est mise en oeuvre dans le document J.N.Wilson, C.G.Bangcuyo, B.Erdogan, M.L.Myrick, U.H.F.Bunz, *Macromolecules,* **36** (2003) 1426-28. Des nanotubes de polyaryleneéthynylenes sont obtenus en faisant passer des flux de polymères au travers de membranes poreuses. Les propriétés d'auto-assemblage des polyaryleneéthynylenes sont utilisées pour former des nanotubes dans les pores des membranes. Ces nanotubes ne présentent pas une forme figée.

### e) Synthèse de nano-objets en copolymères blocs

Les nanotubes de copolymères blocs peuvent être obtenus par associations physiques dudit copolymère bloc dans un solvant ou un mélange de solvants, le solvant ayant la particularité de posséder une affinité préférentielle pour l'un des blocs du copolymère. Cette séparation de phase est en particulier gouvernée par la présence de groupe solubilisant sur ce bloc. Par rapport aux autres procédés de synthèse présentés, la formation des nanotubes passe par une étape de réticulation de l'un des blocs qui permet de figer la forme du nanotube et de conférer une forme et une structure stables quel que soit le milieu dans lequel les nanotubes de polymère sont intégrés.

Le polymère utilisé pour réaliser des nanotubes par cette voie de synthèse peut être un dibloc AB ou un tribloc ABA, BAB ou ABC. Au moins un des blocs A, B ou C doit pouvoir être réticulé. Le bloc réticulé peut être celui formant le coeur du nanotube ou l'enveloppe du nanotube. Les monomères utilisés pour former le bloc réticulé sont choisi parmi ceux ayant des groupements fonctionnels de réticulation de type X ou de type Y. Les liaisons formées étant de type X-X, Y-Y ou X-Y. Les groupements fonctionnels X sont choisis parmi la liste suivante : XHn avec X = O, N, S, COO et n = 1 ou 2, notamment les alcools, amines, thiols et acides carboxyliques. Les groupements fonctionnels Y sont choisis par exemple parmi les fonctions :
- Epoxyde
- Aziridine
- double liaison activée ou non tels que les éthylènes et dérivés comme par exemple le butadiène, l'isoprène, les esters acryliques ou méthacryliques, esters vinyliques, éthers vinyliques, esters cinnamiques, styrènes et dérivés, esters crotoniques et maléiques, anhydrides, chlorures d'acides insaturés, acide crotonique, acide cinnamique
- Aldéhydes
- Acétals, hémi acétals
- Aminals, hémi aminals
- Cétones, alpha-hydroxycétones, alpha-halocétones
- Lactones, thiolactones
- Isocyanate
- Thiocyanate
- Imines
- Imides en particulier succimide et glutimide
- N-hydroxysuccinimide esters
- Imidates
- Thiosulfate
- Oxazine et oxazoline
- Oxazinenium et oxazolinium
- Halogénures d'alkyle en C₁ à C₃₀ ou d'aryle et aralkyle en C₆ à C₃₀ de formule RX avec X = I, Br, Cl
- Halogénure de cycle insaturé, carboné ou hétérocycle, notamment les chlorotriazine, chloropyrimidine, chloroquiinoxaline, chlorobenzotriazole
- Halogénure de sulfonyle : RSO₂Cl ou F, R étant un alkyle en C₁ à C₃₀.

La réticulation peut être effectuée par le biais d'un composé intermédiaire comportant plus de deux fonctions du type Y ou X.

La taille (longueur, diamètre, épaisseur) des nanotubes dépend du choix des blocs, de la taille des blocs, de la proportion des blocs et du choix des solvants.

Les groupements solubilisants sont de préférence choisis dans le groupe formé par :
- un radical carboxylique (-COOH), carboxylate (-COO⁻M⁺, M représentant un métal alcalin comme le sodium, le potassium, un métal alcalino-terreux, une amine organique telle que une amine primaire, secondaire ou ternaire, une alcanolamine, un acide aminé),
- un radical sulfonique (-SO₃H), sulfonate (-SO₃⁻ M⁺, M ayant la même définition que ci dessus),
- un radical amine primaire, secondaire, ternaire,
- un radical ammonium quaternaire tel -NR'₃⁺ Z⁻ avec Z = Br, Cl, alkyl (C₁-C₄)-OSO₃ et R' alkyles identiques ou non, linéaires ou ramifiés en C₁ à C₂₀, ou formant un hétérocycle avec l'azote pour deux d'entre eux,
- un radical hydroxyle,
- un radical polyoxyde d'alcène en C₂-C₃.

Les fonctions acides carboxyliques ou sulfoniques peuvent ou non être neutralisées par une base, telle que l'hydroxyde de sodium, l'amino-2 méthyl-2 propanol, le triéthylamine ou encore le tributylamine.

Les radicaux amines peuvent ou non être neutralisés par un acide minéral, tel que l'acide chlorhydrique, ou par un acide organique, tel que les acides acétique ou lactique, par exemple.

En outre, il est à noter que les dits radicaux solubilisants peuvent être reliés au cycle par l'intermédiaire d'un groupement espaceur tel que par exemple un radical -R", -OR", -OCOR"- ou encore -COOR"- avec R" représentant un radical alkyle, linéaire ou ramifié en C₁-C₂₀, comprenant éventuellement un ou plusieurs hétéroatomes, tels que l'oxygène par exemple.

Selon un mode de réalisation particulier de l'invention, le polymère mis en oeuvre comporte au moins un groupement solubilisant par unité répétitive (monomère).

Des exemples de tels nanotubes sont décrits dans G.Liu, *Handbook of Nanostructured Materials and Technology,* (2000) p 475-500: poly (2-cinnamoylethylmethacrylate) (PCEMA) : PS-*b*-PCEMA (polystyrène-*b*-poly (2-cinnamoylethylmethacrylate)), PAA-*b*-PCEMA (polyacide-acrylate-*b*-poly (2-cinnamoylethylmethacrylate)), PBtA-*b*-PCEMA (polybutylacrylate-*b*-poly (2-cinnamoylethylmethacrylate)), PI-*b*-PCEMA (polyimine-*b*-poly (2-cinnamoylethylmethacrylate)). D'autre exemples préférés sont les copolymères blocs dont une séquence est un polyméthylacrylate de diméthyl-amino-éthyle, ou PAA, ou un polyacide méthacrylique, ou POE, ou silicone, comme par exemple polyméthylacrylate de diméthyl-amino-éthyle-b-cynnamoyl, PAA-b-poly(cynnamoylcoacrylate de butyle ou co styrène ou cométhacrylate de méthyle) silicone-b- cynnamoyl, PS-b-(polyCEMAcoHEMA).

La forme et la taille des tubes dépendent du choix des monomères dans les blocs et de la proportion des blocs dans le polymère bloc. On peut obtenir des tubes creux ou pleins suivant le choix des polymères et des solvants.

Selon la technique utilisée, le nano-objet peut-être plein ou creux.

Dans le cas d'un nano-objet creux, , il peut être fermé en ses extrémités ou ouvert ; en outre il peut contenir un ou plusieurs composés de remplissage qui peuvent être choisis parmi les gaz, les liquides ou les solides, ces composés de remplissage étant différents du polymère constituant le nano-objet.

Les nano-objets peuvent absorber ou adsorber à l'intérieur ou à l'extérieur de leur paroi une ou plusieurs molécules tels que par exemple des polymères linéaires ou ramifiés, des fullerènes dopés ou non, nanotubes de carbone, ou encore des additifs cosmétiques dont la taille est compatible avec les nano-objets.

La surface externe des nano-objets peut également être fonctionnalisée pour améliorer l'affinité des nano-objets avec la fibre kératinique ou avec le milieu.

Par « surface fonctionnalisée » au sens de la présente demande, on entend que la surface externe est modifiée par la présence de groupements fonctionnels pouvant interagir physiquement ou chimiquement entre eux, avec la fibre ou encore avec le milieu dans lequel les nano-objets sont placés.

L'augmentation de l'affinité des nanotubes pour la fibre kératinique se fait notamment au moyen de groupes possédant une certaine réactivité sur les acides aminés constituant la matière kératinique. De préférence, le ou les groupements fonctionnels susceptibles de créer une ou plusieurs liaisons chimiques covalentes avec la fibre kératinique sont choisis parmi les groupements susceptibles de réagir avec les thiols, les disulfures, les acides carboxyliques, les alcools et les amines.

De préférence, la composition cosmétique selon la présente invention comprend de 0,00001 à 30 % en poids et de manière préférée de 0,001 à 10 % en poids de nano-objets de forme allongée en polymère réticulé, par rapport au poids total de la composition.

La composition cosmétique selon la présente invention peut comprendre un ou plusieurs nano-objets de forme allongée c'est-à-dire qu'elle peut comprendre des nano-objets tous constitués du même polymère ou constitués de polymères différents.

Le milieu cosmétiquement acceptable est généralement constitué par de l'eau ou par un ou plusieurs solvants organiques ou par un mélange d'eau et d'un ou plusieurs solvants organiques.

Le ou les solvants organiques peuvent être choisis parmi les solvants suivants : les alcools en C₁-C₄ tel que l'éthanol et l'isopropanol, les alcanes en C₅-C₁₀, les cétones telles que l'acétone et la méthyléthylcétone, les éthers tels que le diméthoxyéthane et le diéthoxyéthane, les esters tels que l'acétate de méthyle, l'acétate d'éthyle, l'acétate de butyle, des alcools gras, des polyols modifiés ou nom tel que le glycérol, des silicones volatiles ou non, des huiles minérales, organiques ou végétales, des cires, des acides gras et leurs mélanges.

Le milieu cosmétiquement acceptable peut se présenter sous forme émulsionnée, les nano-objets peuvent aussi être encapsulés.

La composition cosmétique selon l'invention peut en outre contenir au moins un additif cosmétique.

Par « additif cosmétique » au sens de la présente demande on entend, les agents oxydants, réducteurs, les polymères fixants sous forme soluble, dispersée, micro-dispersée, les polymères épaississants, les agents tensio-actifs non-ioniques, anioniques, cationiques et amphotères, les agents conditionneurs, les agents adoucissants, les agents hydratants, les agents émollients, les agents anti-mousse, les céramides et pseudo-céramides, les vitamines et pro-vitamines dont le panthénol, les corps gras non siliconés tels que les huiles végétales, animales, minérales et synthétiques, les silicones volatiles ou non, linéaires ou ramifiées, organomodifiées ou non, les filtres solaires hydrosolubles et liposolubles, siliconés ou non siliconés, les pigments minéraux et organiques, colorés ou non colorés, les colorants permanents ou temporaires, les charges minérales, les argiles, les minéraux, les colloïdaux, les agents nacrants et opacifiants, les protéines, les agents séquestrants, les agents plastifiants, les agents solubilisants, les agents acidifiants, des agents alcalinisants, les hydroxyacides, les agents de pénétration, les parfums, les agents solubilisants du parfum (peptisants), les agents conservateurs, les agents anti-corrosion.

Ces additifs sont présents dans la composition cosmétique selon l'invention en une quantité allant de 0 à 20 % en poids par rapport au poids total de la composition cosmétique.

L'homme de métier veillera à choisir les éventuels additifs et leur quantité de manière à ce qu'ils ne nuisent pas aux propriétés des compositions de la présente invention.

La composition selon l'invention peut se présenter sous la forme d'une lotion, d'un spray, d'une mousse, d'un shampooing, d'un après-shampooing.

La composition selon l'invention peut également se présenter sous forme de laque, elle est alors appliquée au moyen d'un gaz propulseur. Ce gaz propulseur est constitué par les gaz comprimés ou liquéfiés habituellement employés pour la préparation de composition aérosols.

Un deuxième objet de l'invention est un procédé de traitement cosmétique pour conférer du volume à la coiffure tel qu'on applique sur les fibres kératiniques, en particulier des fibres kératiniques humaines telles que les cheveux la composition selon la présente invention.

Un troisième objet de l'invention est une utilisation sur les fibres kératiniques, en particulier des fibres kératiniques humaines telles que les cheveux de la composition cosmétique selon la présente invention pour conférer du volume à la coiffure.

La composition peut être appliquée sur cheveux secs (produit non rincé), ou sur cheveux mouillés (produit rincé). Dans ce dernier cas le temps d'application sur les cheveux avant rinçage est de quelques secondes à quelques minutes généralement entre 5 secondes et 20 minutes.

Les fibres kératiniques peuvent également être chauffées avant, après ou pendant l'application de la composition selon la présente invention.

Les exemples qui suivent illustrent l'invention sans s'en limiter la portée.

### Exemple 1

### Synthèse des nanotubes de polyacrylate de tertiobutyle (PATB)-b-PCEMA :

L'acrylate de tertiobutyle est polymérisé à -78°C dans le tetrahydrofurane (THF) avec le sec-butyl lithium comme initiateur. Ensuite, le 1,1-diphényl éthylène (DPE) et le chlorure de lithium (3 équivalents en mole de sec-butyl lithium) sont ajoutés. Le DPE réagit avec l'anion polyacrylate de tertiobutyle pour former un anion PATB-DPE. L'autre bloc est préparé en réalisant la polymérisatioon de trimethylsiloxyethyl methacrylate (HEMA-TMS) avec l'anion PATB - DPE. Les groupes trimethylsilyles sont éliminés par hydrolyse en présence de THF/methanol. On obtient ainsi un dibloc PATB - block - poly(2-hydroxylethyl methacrylate (PATB -*b*-PHEMA). Par réaction de chlorure de cinnamoyle en présence de pyridine on obtient le dibloc PATB-*b*-PCEMA. La dernière étape consiste en une hydrolyse dans un mélange eau/acide trifluoroacétique. Le dibloc PATB -*b*-PCEMA a une fraction de PCEMA égale 24% en poids du polymère. La solution est placée dans un bêcher à fond large. Au bout de quatre jours on obtient un film qui est ensuite séché pendant trois jours à 65°C et pendant trois jours à 105°C. Le film est ensuite irradié sous une lampe à mercure (500W) (filtre à 310 nm). Le film ainsi obtenu est ensuite dissous dans l'eau. Les nanotubes obtenus ont un diamètre de 50 nm et une longueur de 20 µm.

Un gramme de la composition 1 selon la présente invention comprenant des nanotubes de PATB-*b*-PCEMA (24 % de PCEMA) est appliqué sur 2,5 g de cheveux européens.

### Composition 1

| | |
|---|---|
| cyclopentasiloxane | 10,0% |
| Huile de ricin hydrogénée oxyéthylénée(70E) | 10,0% |
| Diméthicone copolyol | 0,5 % |
| Propylène glycol | 2.5 % |
| Chlorure de behentrimonium | 1,2 % |
| Nanotubes de PATB-*b*-PCEMA | 0,01 % |
| Eau | 75,79 % |

Après quelques minutes de pose, les cheveux sont rincés à l'eau puis mis en forme au sèche-cheveux.

La composition 1 permet une augmentation nette du volume de la chevelure qui dure plusieurs jours.

### Exemple 2

### Synthèse des nanotubes de polythiophène :

9g de bis(éthylhèxyl) sulfosuccinate de sodium (AOT) sont dissous dans 40 ml d'hexane à température ambiante. L'AOT forme un micelle inverse. 1 ml d'une solution aqueuse de 9M de FeCl₃ est ajouté au mélange AOT/hexane. Le FeCl₃ permet de former des micelles en forme de tiges (il permet de diminuer la concentration critique micellaire secondaire (CMCII) et d'augmenter la force ionique du solvant. Le groupe anionique polaire de AOT extrait le cation métallique de la phase aqueuse. 0,5g de monomère de thiophène est ajouté dans le solvant. Le monomère de thiophène polymérise sur la face externe des micelles pendant trois heures. En ajoutant du solvant (éthanol) et en laissant précipiter la solution pendant deux heures, on élimine le tensio-actif.

Un gramme de la composition 2 selon la présente invention comprenant des nanotubes de polythiophène est appliqué sur 2,5 g de cheveux européens.

### Composition 2

| | |
|---|---|
| cyclopentasiloxane | 10,0% |
| Huile de ricin hydrogénée oxyéthylénée (7OE) | 10,0% |
| Diméthicone copolyol | 0,5 % |
| Propylène glycol | 2,5 % |
| Chlorure de behentrimonium | 1,2 % |
| Nanotubes de polythiophène | 0,01 % |
| Eau | 75,79 % |

Après quelques minutes de pose, les cheveux sont rincés à l'eau puis mis en forme au sèche-cheveux.

La composition 2 permet une augmentation nette du volume de la chevelure qui dure plusieurs jours.

## Revendications

1. Composition cosmétique pour le traitement cosmétique des fibres kératiniques, en particulier des fibres kératiniques humaines telles que les cheveux, comprenant dans un milieu cosmétiquement acceptable, au moins un nano-objet de forme allongée en polymère synthétique réticulé et au moins un additif cosmétique choisi dans le groupe formé par les agents oxydants, réducteurs, les polymères fixants sous forme soluble, dispersée, micro-dispersée, les polymères épaississants, les agents tensio-actifs non-ioniques, anioniques, cationiques et amphotères, les agents conditionneurs, les agents adoucissants, les agents hydratants, les agents émollients, les agents anti-mousse, les céramides et pseudo-céramides, les vitamines et pro-vitamines dont le panthénol, les corps gras non siliconés tels que les huiles végétales, animales, minérales et synthétiques, les silicones volatiles ou non, linéaires ou ramifiées, organomodifiées ou non, les filtres solaires hydrosolubles et liposolubles, siliconés ou non siliconés, les pigments minéraux et organiques, colorés ou non colorés, les colorants permanents ou temporaires, les charges minérales, les argiles, les minéraux, les colloïdaux, les agents nacrants et opacifiants, les protéines, les agents séquestrants, les agents plastifiants, les agents acidifiants, des agents alcalinisants, les hydroxyacides, les agents de pénétration, les parfums, les agents conservateurs, les agents anti-corrosion.

2. Composition selon la revendication 1 telle que le nano-objet de forme allongée est un nanotube.

3. Composition selon la revendication 2 telle que le nanotube présente une section circulaire ou elliptique de diamètres externes ou d'axes compris entre 0,1 et 200 nm.

4. Composition selon la revendication 2 telle que le nanotube présente une section hexagonale de diamètre externe compris entre 0,1 et 200 nm.

5. Composition selon la revendication 1 telle que le nano-objet est une hélice de diamètre externe compris entre 0,1 et 200 nm.

6. Composition selon l'une des revendications précédentes telle que le nanotube présente une longueur comprise entre 10 nm et 10µm.

7. Composition selon l'une des revendications précédentes telle que le rapport entre la longueur et la section du nano-objet est strictement supérieur à 1, de préférence supérieur à 2 et de manière encore plus préférée supérieur à 3, ce rapport étant de préférence inférieur à 100 000.

8. Composition selon l'une des revendications précédentes telle que le polymère synthétique est obtenu par synthèse chimique ou électrochimique telles que la polymérisation radicalaire, polycondensation, polymérisation par ouverture de cycle, polymérisation par métathèse.

9. Composition selon l'une des revendications précédentes telle que le polymère synthétique réticulé est un homopolymère.

10. Composition selon la revendication 9 telle que l'homopolymère est choisi dans le groupe formé par le polypyrrole, le polyaniline, le polyéthylènedioxythiophène, le polyméthylméthacrylate, le polytétrafluoroéthylène, le poly-L-lactide/palladium-acétate, le poly(p-xylène), le [2.2]paracyclophane polymérisé, le 1,4-quinodiméthane polymérisé, le polystyrène, le polypropylène, le polyaryléthylène, le poly(p-phénylène benzo-bis-oxazole).

11. Composition selon l'une des revendications 1 à 8 telle que le polymère synthétique réticulé est un copolymère.

12. Composition selon la revendication 11 telle que le copolymère est un copolymère bloc.

13. Composition selon la revendication 11 ou 12 telle que le copolymère est choisi dans le groupe formé par les polystyrène-*b*-poly(2-cinnamoylethylmethacrylate), polyacideacrylique-*b-*poly(2-cinnamoylethylmethacrylate), polybutylacrylate-*b-*poly(2-cinnamoyl-ethylmethacrylate), polyimine-*b*-poly(2-cinnamoylethylmethacrylate).

14. Composition selon l'une des revendications précédentes telle que le nano-objet est creux.

15. Composition selon la revendication 14 telle que le nano-objet contient un ou plusieurs composés de remplissage choisis parmi les gaz, les liquides ou les solides.

16. Composition selon l'une des revendications précédentes telle qu'une ou plusieurs molécules sont absorbées ou adsorbées à l'intérieur ou à l'extérieur de la paroi du nano-objet.

17. Composition selon l'une des revendications précédentes telle que la surface externe du nano-objet est fonctionnalisée.

18. Composition selon l'une des revendications précédentes telle que le nano-objet est plein.

19. Composition selon l'une des revendications précédentes telle que la concentration en nano-objets de forme allongée est comprise entre 0,00001 et 30% et de préférence entre 0,0001 et 10% en poids par rapport au poids total de la composition.

20. Composition selon l'une des revendications précédentes telle que le milieu cosmétiquement acceptable comprend au moins un solvant choisi dans le groupe formé par les alcools en C₁-C₄ tel que l'éthanol et l'isopropanol, les alcanes en C₅-C₁₀, les cétones telles que l'acétone et la méthyléthylcétone, les éthers tels que le diméthoxyéthane et le diéthoxyéthane, les esters tels que l'acétate de méthyle, l'acétate d'éthyle, l'acétate de butyle, des alcools gras, des polyols modifiés ou nom tel que le glycérol, des silicones volatiles ou non, des huiles minérales, organiques ou végétales, des cires, des acides gras et leurs mélanges.

21. Procédé de traitement cosmétique pour conférer du volume à la coiffure **caractérisé en ce que** l'on applique sur les fibres kératiniques, en particulier des fibres kératiniques humaines telles que les cheveux une composition selon l'une des revendications précédentes.

22. Utilisation sur les fibres kératiniques, en particulier des fibres kératiniques humaines telles que les cheveux de la composition cosmétique selon l'une des revendications 1 à 20 pour conférer du volume à la coiffure.

## Claims

1. Cosmetic composition for cosmetic treatment of keratin fibres, especially human keratin fibres such as the hair, which comprises, in a cosmetically acceptable medium, at least one nanoobject of elongate form made of crosslinked synthetic polymer, and comprising at least one cosmetic additive selected from the group consisting of oxidizing agents; reducing agents; fixative polymers in soluble, dispersed or microdispersed form; thickening polymers; nonionic, anionic, cationic and amphoteric surfactants; conditioning agents; softeners; moisturizers; emollients; antifoams; ceramides and pseudoceramides; vitamins and provitamins, including panthenol; non-silicone fats such as vegetable, animal, mineral and synthetic oils; volatile or nonvolatile, linear or branched silicones, with or without organic modification; water-soluble and fat-soluble, silicone or non-silicone sunscreens; organic and inorganic pigments, coloured or non-coloured; permanent or temporary dyes; mineral fillers; clays; minerals; colloids; nacres and opacifiers; proteins; sequestrants; plasticizers; acidifying agents; alkalifying agents; hydroxy acids; penetrants; perfumes; preservatives and anti-corrosion agents.

2. Composition according to Claim 1, wherein the nanoobject of elongate form is a nanotube.

3. Composition according to Claim 2, wherein the nanotube has a circular or ellipsoidal cross-section with external diameters or axes of between 0.1 and 200 nm.

4. Composition according to Claim 2, wherein the nanotube has a hexagonal cross-section with an external diameter of between 0.1 and 200 nm.

5. Composition according to Claim 1, wherein the nanoobject is a helix with an external diameter of between 0.1 and 200 nm.

6. Composition according to one of the preceding claims, wherein the nanotube has a length of between 10 nm and 10µm.

7. Composition according to one of the preceding claims, wherein the ratio between the length and the cross-section of the nanoobject is strictly greater than 1, preferably greater than 2 and more preferably greater than 3, this ratio being preferably less than 100 000.

8. Composition according to one of the preceding claims, wherein the synthetic polymer is obtained by chemical or electrochemical synthesis such as free-radical addition polymerization, polycondensation, ring-opening polymerization or metathesis polymerization.

9. Composition according to one of the preceding claims, wherein the crosslinked synthetic polymer is a homopolymer.

10. Composition according to Claim 9, wherein the homopolymer is selected from the group consisting of polypyrrole, polyaniline, polyethylenedioxythiophene, polymethyl methacrylate, polytetraflurorethylene, poly-L-lactide/palladium acetate, poly(p-xylene), polymerized [2.2]paracyclophane, polymerized 1,4-quinodimethane, polystyrene, polypropylene, polyarylethylene and poly(p-phenylenebenzobisoxazole).

11. Composition according to one of Claims 1 to 8, wherein the crosslinked synthetic polymer is a copolymer.

12. Composition according to Claim 11, wherein the copolymer is a block copolymer.

13. Composition according to Claim 11 or 12, wherein the copolymer is selected from the group consisting of polystyrene-*b*-poly(2-cinnamoylethyl methacrylate)s, polyacrylic acid-*b*-poly(2-cinnamoylethyl methacrylate)s, polybutylacrylate-b-poly(2-cinnamoyl-ethyl methacrylate)s and polyimine-*b-*poly(2-cinnamoylethyl methacrylate)s.

14. Composition according to one of the preceding claims, wherein the nanoobject is hollow.

15. Composition according to Claim 14, wherein the nanoobject contains one or more filling compounds selected from gases, liquids and solids.

16. Composition according to one of the preceding claims, wherein one or more molecules is or are absorbed or adsorbed on the inside or the outside of the wall of the nanoobject.

17. Composition according to one of the preceding claims, wherein the outside surface of the nanoobject is functionalized.

18. Composition according to one of the preceding claims, wherein the nanoobject is non hollow.

19. Composition according to one of the preceding claims, wherein the concentration of nanoobjects of elongate form is between 0.00001% and 30% and preferably between 0.0001% and 10% by weight, relative to the total weight of the composition.

20. Composition according to one of the preceding claims, wherein the cosmetically acceptable medium comprises at least one solvent selected from the group consisting of C₁-C₄ alcohols such as ethanol and isopropanol, C₅-C₁₀ alkanes, ketones such as acetone and methyl ethyl ketone, ethers such as dimethoxyethane and diethoxyethane, esters such as methyl acetate, ethyl acetate and butyl acetate, fatty alcohols, modified or non-modified polyols such as glycerol, volatile or nonvolatile silicones, mineral, organic or vegetable oils, waxes, fatty acids and mixtures thereof.

21. Method of cosmetic treatment for imparting body to the hairstyle, **characterized in that** a composition according to one of the preceding claims is applied to keratin fibres, especially human keratin fibres such as the hair.

22. Use of the cosmetic composition according to one of Claims 1 to 20 on keratin fibres, especially human keratin fibres such as the hair, for the purpose of imparting body to the hairstyle.

## Patentansprüche

1. Kosmetische Zusammensetzung für die kosmetische Behandlung von Keratinfasern, insbesondere menschlichen Keratinfasern, wie Haaren, die in einem kosmetisch akzeptablen Medium mindestens einen Nanogegenstand von langgestreckter Form aus einem vernetzten synthetischen Polymer und mindestens einen kosmetischen Zusatzstoff enthält, der unter den Oxidationsmitteln, Reduktionsmitteln, fixierenden Polymeren in löslicher, dispergierter, mikrodispergierter Form, verdickenden Polymeren, nichtionischen, anionischen, kationischen und amphoteren grenzflächenaktiven Stoffen, Konditioniermitteln, Mitteln für die Geschmeidigkeit, Hydratisierungsmitteln, Emollientien, Schaumverhütungsmitteln, Ceramiden und Pseudoceramiden, Vitaminen und Provitaminen, darunter Panthenol, nicht siliconierten Fettsubstanzen, wie pflanzlichen Ölen, tierischen Ölen, Mineralölen und synthetischen Ölen, flüchtigen oder nicht flüchtigen, linearen oder verzweigten, organomodifizierten oder nicht organomodifizierten Siliconen, wasserlöslichen und fettlöslichen, siliconierten oder nicht siliconierten Sonnenschutzfiltern, farbigen oder nicht farbigen, anorganischen und organischen Pigmenten, permanenten oder temporären Farbstoffen, anorganischen Füllstoffen, Tonen, Mineralien, Kolloiden, Perlglanzstoffen und Trübungsmitteln, Proteinen, Maskierungsmitteln, Weichmachern, Ansäuerungsmitteln, Alkalisierungsmitteln, Hydroxysäuren, Penetrationsmitteln, Parfums, Konservierungsstoffen und Korrosionsschutzmitteln ausgewählt ist.

2. Zusammensetzung nach Anspruch 1, wobei der Nanogegenstand länglicher Form eine Nanoröhre ist.

3. Zusammensetzung nach Anspruch 2, wobei die Nanoröhre einen runden oder elliptischen Querschnitt mit einem Außendurchmesser oder Achsen im Bereich von 0, 1 bis 200 nm aufweist.

4. Zusammensetzung nach Anspruch 2, wobei die Nanoröhre einen hexagonalen Querschnitt mit einem Außendurchmesser von 0,1 bis 200 nm aufweist.

5. Zusammensetzung nach Anspruch 1, wobei der Nanogegenstand eine Helix mit einem Außendurchmesser von 0,1 bis 200 nm ist.

6. Zusammensetzung nach einem der vorhergehenden Ansprüche, wobei die Nanoröhre eine Länge von 10 nm bis 10 µm aufweist.

7. Zusammensetzung nach einem der vorhergehenden Ansprüche, wobei das Verhältnis der Länge und des Querschnitts des Nanogegenstandes immer über 1, vorzugsweise über 2 und noch bevorzugter über 3 liegt, wobei das Verhältnis vorzugsweise unter 100 000 liegt.

8. Zusammensetzung nach einem der vorhergehenden Ansprüche, wobei das synthetische Polymer durch chemische oder elektrochemische Synthese hergestellt wird, wie radikalische Polymerisation, Polykondensation, Polymerisation unter Ringöffnung, Metathese-Polymerisation.

9. Zusammensetzung nach einem der vorhergehenden Ansprüche, wobei das vernetzte synthetische Polymer ein Homopolymer ist.

10. Zusammensetzung nach Anspruch 9, wobei das Homopolymer unter Polypyrrol, Polyanilin, Polyethylendioxythiophen, Polymethylmethacrylat, Polytetrafluorethylen, Poly-L-lactid/Palladiumacetat, Poly(p-xylol), polymerisiertem [2.2]Paracyclophan, polymerisiertem 1,4-Chinodimethan, Polystyrol, Polypropylen, Polyarylethylen, Poly(p-phenylen-benzo-bis-oxazol) ausgewählt ist.

11. Zusammensetzung nach einem der Ansprüche 1 bis 8, wobei das vernetzte synthetische Polymer ein Copolymer ist.

12. Zusammensetzung nach Anspruch 11, wobei das Copolymer ein Blockcopolymer ist.

13. Zusammensetzung nach Anspruch 11 oder 12, wobei das Copolymer unter Polystyrol-b-poly(2-cinnamoylethylmethacrylat), Polyacrylsäure-b-poly(2-cinnamoylethylmethacrylat), Polybutylacrylat-b-poly(2-cinnamoylethylmethacrylat), Polyimin-*b*-poly(2-cinnamoylethylmethacrlyat) ausgewählt ist.

14. Zusammensetzung nach einem der vorhergehenden Ansprüche, wobei der Nanogegenstand hohl ist.

15. Zusammensetzung nach Anspruch 14, wobei der Nanogegenstand eine oder mehrere Verbindungen zur Füllung enthält, die unter Gasen, Flüssigkeiten oder Feststoffen ausgewählt sind.

16. Zusammensetzung nach einem der vorhergehenden Ansprüche, wobei ein oder mehrere Moleküle innen oder außen an der Wand des Nanogegenstands absorbiert oder adsorbiert sind.

17. Zusammensetzung nach einem der vorhergehenden Ansprüche, wobei die äußere Oberfläche des Nanogegenstands funktionalisiert ist.

18. Zusammensetzung nach einem der vorhergehenden Ansprüche, wobei der Nanogegenstand ein Vollmaterial ist.

19. Zusammensetzung nach einem der vorhergehenden Ansprüche, wobei die Konzentration der Nanogegenstände von langgestreckter Form im Bereich von 0,00001 bis 30 % und vorzugsweise 0,0001 bis 10 Gew.-%, bezogen auf das Gesamtgewicht der Zusammensetzung, liegt.

20. Zusammensetzung nach einem der vorhergehenden Ansprüche, wobei das kosmetisch akzeptable Medium mindestens ein Lösungsmittel enthält, dass unter den C₁-₄-Alkoholen, wie Ethanol und Isopropanol, C₅₋₁₀-Alkanen, Ketonen, wie Aceton und Methylethylketon, Ethern, wie Dimethoxyethan und Diethoxyethan, Estern, wie Methylacetat, Ethylacetat, Butylacetat, Fettalkoholen, modifizierten oder nichtmodifizierten Polyolen, wie Glycerin, flüchtigen oder nicht flüchtigen Siliconen, Mineralölen, organischen oder pflanzlichen Ölen, Wachsen, Fettsäuren und deren Gemischen ausgewählt ist.

21. Verfahren zur kosmetischen Behandlung, um der Frisur Volumen zu geben, **dadurch gekennzeichnet, dass** auf die Keratinfasern und insbesondere menschliche Keratinfasern, wie Haare, eine Zusammensetzung nach einem der vorhergehenden Ansprüche aufgetragen wird.

22. Verwendung der kosmetischen Zusammensetzung nach einem der Ansprüche 1 bis 20 an Keratinfasern, insbesondere menschlichen Keratinfasern, wie Haaren, um der Frisur Volumen zu geben.
